# EUROPEAN PATENT APPLICATION

(11) **EP 1 310 815 A2**
(43) Date of publication of application: **14.05.2003**
(21) Application number: 02254098.3
(22) Date of filing: 12.06.2002
(51) Int. Cl.: G02B 6/44

(54) **Fiberoptic coil tray and carrier package**

(30) Priority: 13.06.2001 US 880258
(71) Applicant: ETHICON ENDO-SURGERY, INC., Cincinnati, Ohio 45242 (US)
(72) Inventor: Sheetz, Jane, Cincinnati, OH 45241 (US); Daugird, Joanne, Deer Park, NY 11729 (US); Heaton, Leslie A., Fairfield, OH 45014 (US); Kolata, Ronald J., Cincinnati, OH 45249 (US); Kraimer, William J., Mason, OH 45040 (US); Paddock, Lauren H., Loveland, OH 45140 (US); Rhad, Edward, Fairfield, OH 45014 (US); Kuhns, Jesse, Cincinnati, OH 45330 (US); Frederickson, Scott, Evansville, IN 53536 (US); Wakevainen, Gary T., Loveland, OH 45140 (US)
(74) Representative: Fisher, Adrian John

(57) **Abstract**

A fiberoptic cable coil tray and coil carrier package comprises a carrier to which the fiberoptic coil is attached and which is inserted into the tray, and a lid for the tray. A plurality of fiberoptic coil retainers in the form of notches or clips are formed on the coil carrier to provide a stable, self-contained and restrained fiberoptic coil which remains properly seated in the package during handling and storage. The coil carrier provides increased ease of handling of the fiberoptic coil by restraining individual fiberoptic coils with the plurality of coil retainers which are positioned along the length of the fiberoptic cable, such that a selected length of the fiberoptic cable can be removed from the carrier without the remaining coils springing out therefrom. The carrier also secures each end of the fiberoptic cable, such that either end can be removed separately while the remainder of the fiberoptic coils remain secured to the carrier.

## Description

### 1. Field of the Invention

The present invention relates generally to a fiberoptic coil tray and carrier package, and more particularly pertains to a fiberoptic cable coil tray and carrier package, and a method of packaging a fiberoptic coil, wherein a plurality of fiberoptic coil retainer notches or clips are formed on select portions of the carrier to provide a stable, self-contained and restrained coil which remains properly seated in the package during handling and storage. The carrier provides increased ease of handling of the fiberoptic coil by restraining the fiberoptic coil with the plurality of separate retainers which are positioned along the length of the fiberoptic coil until it is used, such that a selected length of the fiberoptic coil can be removed from the carrier without the remaining coils springing out therefrom.

The fiberoptic coil package consists of a tray, a carrier to which the fiberoptic coil is attached and which is inserted into the tray, and a lid for the tray. The carrier coils the fiberoptic cable and secures each end thereof, such that either end can be removed separately while the remainder of the fiberoptic coils remain secured to the carrier. Features of the tray and carrier restrain the coiled fiberoptic cable to provide for easier handling of the coiled fiberoptic cable and to decrease the likelihood of the coiled fiberoptic cable springing out when the package is opened.

### 2. Discussion of the Prior Art

A fiberoptic cable is one component of a medical device which must be handled gently and in a sterile fashion throughout a surgical procedure. The fiberoptic cable is generally packaged and shipped as a coil. The entire length of the coil must be controlled to avoid contamination, and the diffuser end of the fiberoptic must be handled with caution to avoid breakage. The length of the coiled fiberoptic cable (8-10 feet) and the spring-like tendency of the fiberoptic coil create problems with control and handling of the fiberoptic coil. Additional limitations on the packaging include the need to maintain a minimum bend radius of the fiberoptic coil and the need to provide EtO (ethylene oxide) sterilization rather than sterilization by gamma irradiation.

The current fiberoptic product is packaged as a multi-loop coil held together with twist-ties. The coil is placed in a protective tray with a snap-on lid. The connector end and the tip end of the coil are held at different corners of the tray via snap-lock retainers. The entire assembly is contained within a flexible sterile pouch.

To unpackage the current fiberoptic coil, the surgeon/user removes the fiberoptic coil from the tray, removes the nylon clips from the coil, hands one end to a technician, and threads the opposite end into an endoscope. However, the entire length of the fiberoptic coil must be controlled by hand as soon as the nylon clips are removed.

The current fiberoptic coil package, while useful for holding the fiberoptic coil, is cumbersome, and makes it difficult for the user to remove the fiberoptic coil. The current package has two pieces, a lid and a tray. The lid is removed, and the fiberoptic coil is disassembled from the tray and is then uncoiled. The current fiberoptic coil packaging (tray lid with straight crimped-on tip protector) makes the fiberoptic coil difficult to remove.

### SUMMARY OF THE INVENTION

Accordingly, it is a primary object of the present invention to provide a fiberoptic coil tray and carrier package, and a method of packaging a fiberoptic coil, which provides a stable, self-contained and restrained fiberoptic coil without the need for nylon cable ties, and without the risk of the fiberoptic coil springing open out of the package as it is being opened.

The subject invention makes unpacking of the fiberoptic coil easier by maintaining a more stable coil with a plurality of integral parts, rather than with external cable ties as in the prior art, and makes it easier to remove a fiberoptic coil in its wound state.

The fiberoptic coil package comprises a tray, a carrier to which the fiberoptic coil is attached and which is inserted into the tray, and a lid for the tray. The carrier coils the fiberoptic cable and secures each end thereof, such that either end can be removed separately while the remainder of the fiberoptic coil remains in a coil on the carrier. The carrier has an attachment such as a clip which may be used to attach the assembled carrier and fiberoptic coil to a surgical drape on a patient being treated. The carrier is sterilized and packaged in a blister tray with a lid such as a Tyvek lid.

The present invention provides an improved package for the next generation of fiberoptic coil with an improved tip protector, wherein the fiberoptic coil consistently remains properly seated in the package tray during shipment. Molded features of the carrier restrain the coiled fiberoptic cable to greatly decrease the likelihood of the tip springing out when the lid is removed from the tray.

Thus, in a first aspect, the present invention provides a packaging arrangement for a coil of fiberoptic cable which includes a plurality of individual coil loops, comprising:
a. an outer packaging tray; and
b. a fiberoptic coil carrier which is inserted into the outer packaging tray for shipment or storage, and which can be removed from the tray, wherein the carrier provides increased ease of handling of the fiberoptic coil by retaining it with a plurality of separate retainers along the length of the fiberoptic cable, such that a selected length of the fiberoptic cable can be removed from the carrier and remaining coils of the fiberoptic cable remain secured to the carrier.

Preferably, the carrier also defines a connector end retainer for retaining a connector end of the fiberoptic cable, and a treatment end retainer for retaining a treatment end of the fiberoptic cable.

Preferably, the outer packaging tray is sealed with a top closure, wherein the closure-sealed tray provides for sterilization of the carrier/fiberoptic coil assembly in the outer packaging tray.

Preferably, the carrier is designed and contoured specific to a particular surgical device, and is configured to retain the particular surgical device until it is removed for usage.

Preferably, the outer packaging tray is generic to a plurality of specific carriers and is not specific to a particular carrier for a particular surgical device, such that it can package a standard fiberoptic coil carrier.

Preferably, the carrier includes an attachment means for attaching the carrier to a support, such that a surgeon can position the carrier conveniently to require a minimum of handling.

The attachment means may comprise a spring clip.

Alternatively, the attachment means may comprise an adhesive area.

Preferably, the carrier is formed from molded plastic, and includes a plurality of molded individual coil loop retainers, each of which retains and secures a single coil loop of the fiberoptic cable, which allows each loop to be individually released to eliminate springing, a molded retainer to retain and secure a distal tip of the fiberoptic cable, and a molded retainer to retain and secure a connector handle of the fiberoptic cable.

Preferably, each individual coil loop retainer is formed by a molded groove.

Preferably, each molded groove defines a pair of opposed undercut shoulders which snap around an inserted individual coil loop.

Preferably, a first recess defines a tip receiver/protector, and a second recess defines a connector handle receiver/protector.

Preferably, the outer packaging tray comprises a rectangular tray which is thermoformed from plastic, the tray has a bottom surface, sidewalls, and a flange at the top of and extending around the sidewalls, and the bottom surface is generally flat with shaped relief areas defining one or more depressions to receive a shaped fiberoptic coil carrier.

Preferably, the relief areas accommodate larger components of the fiberoptic cable such as the connector handle, and also provide sufficient room and clearance to allow fingers to grasp and remove the carrier, and wherein the carrier and fiberoptic coils are supported by intermediate-height plateau surfaces, with the relief areas being positioned below the plateau surfaces.

Preferably, raised studs rise above the plateau surfaces to maintain the carrier and fiberoptic coil in position within the tray, and also provide support for a top closure lid which is sealed to a flange extending around the upper perimeter of the sidewalls.

Preferably, at least one flange corner is recessed to provide an unsealed corner piece of the top closure lid which is suitable for grasping to pry the lid away from the tray.

The carrier may be generally flat, and thermoformed from plastic, and have an exterior profile and shape to fit within the sidewalls and studs and on top of the plateau surfaces of the tray.

The carrier may have an I shape.

The carrier may have a Y shape.

Preferably, the top of the carrier defines a plurality of molded individual coil retainer undercut grooves, an end tip receiver/protector undercut groove, and a connector handle receiver/protector which defines an undercut depression surrounded by raised ridges to retain the connector handle therein.

Preferably, a connector end of the fiberoptic cable is seated in a top portion of the carrier in a recess which is shaped to match the profile of the connector, two opposed thermoformed posts have a negative profile to match a circular barrel of the connector to retain the connector barrel in place therein, the carrier retains individual fiberoptic coils with multiple snap-fit recesses, and one recess has a larger size to secure therein a tip protector at the treatment end of the fiberoptic cable.

Preferably, each snap-fit recess is defined by a series of three offsets which have a profile to match the profile of a fiberoptic cable.

In another aspect, the present invention provides a method of packaging a coil of fiberoptic cable which includes a plurality of individual coil loops, comprising:
a. mounting the fiberoptic coil on a fiberoptic coil carrier which secures the fiberoptic coil to the carrier with a plurality of separate retainers along the length of the fiberoptic cable, such that a selected length of the fiberoptic cable can be removed from the carrier and remaining coils of the fiberoptic cable remain secured to the carrier;
b. packaging the fiberoptic coil carrier with the fiberoptic coil mounted thereon in an outer packaging tray.

Preferably, the method further includes securing a connector end of the fiberoptic cable to the carrier with a connector end retainer on the carrier, and securing a treatment end of the fiberoptic cable to the carrier with a treatment end retainer on the carrier.

Preferably, the method further includes sealing the outer packaging tray with a top closure, and sterilizing the carrier/fiberoptic coil assembly in the closure-sealed outer packaging tray.

Preferably, the method further includes designing and contouring the carrier to be specific to a particular surgical device, and designing and contouring the outer packaging tray to be generic to a plurality of specific carriers, such that the outer packaging tray can package a standard fiberoptic coil carrier.

Preferably, the method further includes securing the fiberoptic coil to the carrier with a plurality of individual coil loop retainers which are molded in the carrier, each of which retains and secures a single coil loop of the fiberoptic coil, which allows each individual coil loop to be individually released from the carrier.

Preferably, the method further includes securing each individual coil loop in a molded groove on the carrier.

Preferably, the method further includes securing each individual coil in a molded groove on the carrier defined by a pair of opposed undercut shoulders which snap around an inserted individual coil loop.

Preferably, the method further includes securing a treatment end of the fiberoptic cable in a first molded recess in the carrier defining a treatment end receiver/protector, and securing a connector end of the fiberoptic cable in a second recess in the carrier defining a connector end receiver/protector.

Preferably, the method further includes packaging the fiberoptic carrier in a rectangular outer packaging tray which has a bottom surface, sidewalls, and a top flange extending around the sidewalls, wherein the bottom surface is generally flat with shaped relief areas defining one or more depressions to receive and support the fiberoptic coil carrier.

Preferably, the method further includes supporting the carrier and fiberoptic coil on intermediate-height plateau surfaces positioned above the relief areas.

Preferably, the method further includes maintaining the carrier and fiberoptic coil in position within the tray by raised studs which rise above the plateau surfaces.

Preferably, the method further includes sealing a top closure lid to the top flange.

### BRIEF DESCRIPTION OF THE DRAWINGS

The foregoing objects and advantages of the present invention for a fiberoptic coil tray and carrier package may be more readily understood by one skilled in the art with reference being had to the following detailed description of several preferred embodiments thereof, taken in conjunction with the accompanying drawings wherein like elements are designated by identical reference numerals throughout the several views, and in which:
Figures 1 and 1A are respectively a perspective view and a side elevational view of related embodiments of packaging spring clips designed to secure and package a coil of fiberoptic cable.
Figure 2 is a side elevational view of a cross-shaped carrier designed to secure and package a coil of fiberoptic cable, and Figure 2A is a sectional view along arrows A-A in Figure 2.
Figure 3 is a perspective illustration of an embodiment of a packaging clip designed to secure and package a coil of fiberoptic cable, and having a spring clip at the top thereof to clip to a drape.
Figure 3A illustrates a variation of the embodiment of Figure 3 which has an adhesive backing strip to attach the packaging clip to a drape, laser, table, etc.
Figures 4, 4A and 4B illustrate a blister type of package for a laser fiberoptic, wherein Figure 4 is a top planar view showing a fiberoptic coil secured in the blister package, Figure 4A is a sectional view along arrows A-A in Figure 4, and Figure 4B is a perspective view of just the fiberoptic coil carrier.
Figure 5 is a side elevational view of a further embodiment of a triad, three-armed embodiment of a fiberoptic coil carrier designed to secure and package a coil of fiberoptic cable.
Figures 6 and 6A are respectively a top planar view and a side sectional view taken along arrows A-A in Figure 6 of a packaging carrier designed to secure and package a coil of fiberoptic cable and having a raised peripheral lip therearound.
Figure 7 is an exploded view of a preferred embodiment of a combined packaging tray and fiberoptic coil carrier designed to secure and package a coil of fiberoptic cable, wherein the top portion of Figure 7 illustrates a separated Tyvek lid, the middle portion of Figure 7 illustrates just the fiberoptic coil carrier securing a coil of fiberoptic cable thereto, and the bottom portion of Figure 7 illustrates just the packaging tray.
Figure 8 is a perspective view of just the fiberoptic coil carrier as shown in the middle of Figure 7 but without a fiberoptic coil positioned thereon and without a spring clip thereon, and illustrates more clearly the molded contour of the carrier.

### DETAILED DESCRIPTION OF THE DRAWINGS

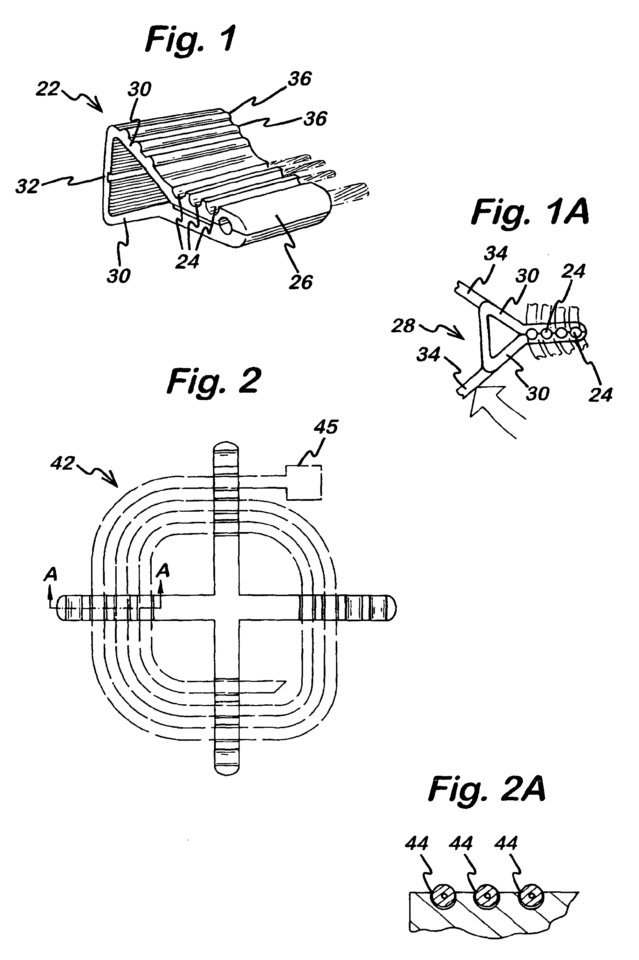

Figures 1 and 1A are respectively a perspective view and a side elevational view of related embodiments 22,28 of packaging spring clips designed to secure and package a coil of fiberoptic cable. Each embodiment includes a binder spring type clip 30, and can further include a living hinge 32, and winged extensions 34 which allow the clip to be opened, traction ribs 36, a plurality of individual tracks 24 for individual coils, with the fiberoptic cable tip being stored in an enclosed tip protector 26. Each track clip 24 secures a single loop which allows each loop to be individually released to eliminate springing out of the remaining loops. The clip 22 can hold the fiberoptic coil in a blister pack and can be used to attach the fiberoptic coil to a drape.

Figure 2 is a side elevational view of a cross or star shaped, preferably thermoformed, carrier 42 designed to secure and package a coil of fiberoptic cable, and Figure 2A is a sectional view along arrows A-A in Figure 2 which shows a minimal undercut 44 in each track 46 in an arm of the carrier. The fiberoptic cable connector handle 45 can be secured in the outermost position by a similar undercut track. The fiberoptic coils are wrapped around and pressed into the undercuts 44 in the star carrier 42, which can then be placed in and sealed in flex packaging. The star carrier 42 can be subsequently removed therefrom, and a selected length of the fiberoptic cable removed from the carrier. The star carrier 42 could also take a triad form, and can also be used with a tray as described hereinbelow.

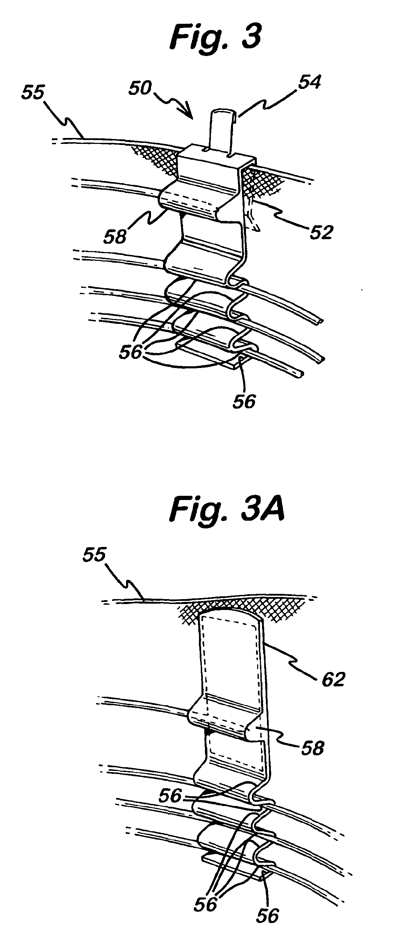

Figure 3 is a perspective illustration of a further embodiment of a packaging clip 50 designed to secure and package a coil of fiberoptic cable, and having a binder clip 52 and a winged extension 54 at the top thereof to clip to a surgical drape 55, and includes three individual coil retention channels 56 and a top closed tip protector 58.

Figure 3A illustrates a variation 60 of the embodiment of Figure 3 which has an adhesive backing strip 62 to attach the packaging clip to a drape, laser, table, etc.

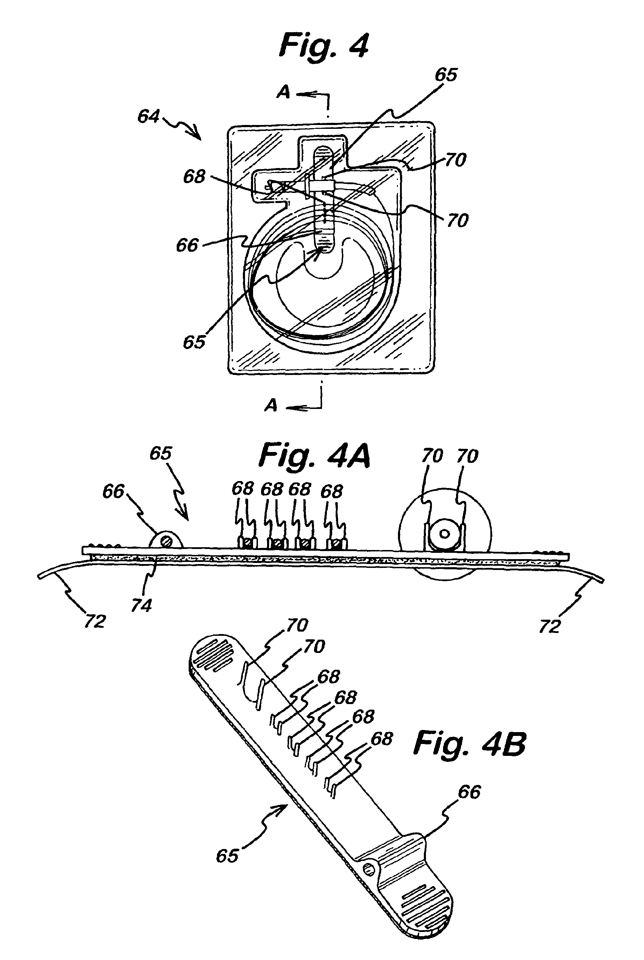

Figures 4, 4A and 4B illustrate a blister type of package 64 for a fiberoptic coil, wherein Figure 4 is a top planar view showing a coil of fiberoptic cable secured within the blister package, Figure 4A is a sectional view along arrows A-A in Figure 4 and shows a fiberoptic coil/carrier 65 without the package which includes a tip retainer/protector 66, a series of four individual clip type coil retainers 68, a connector handle retainer/protector 70, and a pull tab 72 to uncover an adhesive strip 74 to attach the carrier to a drape, etc. Figure 4B is a perspective view of just the fiberoptic coil carrier 65.

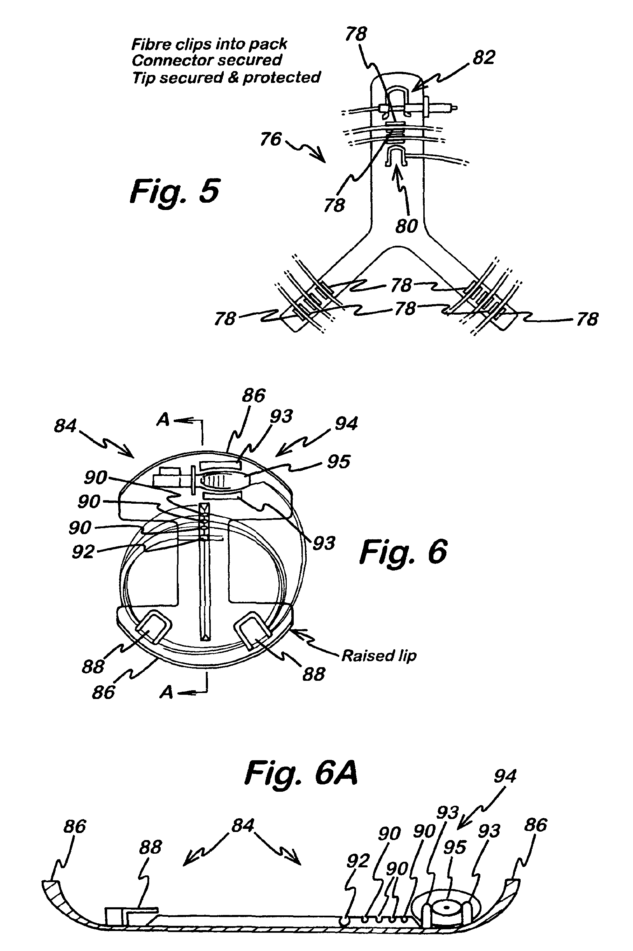

Figure 5 is a side elevational view of a of a triad, three-armed embodiment of a fiberoptic coil carrier 76 designed to secure and package a coil of fiberoptic cable, and having a plurality of individual coil retainer tracks 78, a vertically-extending slot defining a tip receiver/protector 80, and a vertically-extending slot defining a connector handle receiver/protector 82.

Figures 6 and 6A are respectively a top planar view and a side sectional view taken along arrows A-A in Figure 6 of a packaging carrier 84 designed to secure and package a coil of fiberoptic cable and having a raised peripheral lip 86 therearound, two group clips 88, each of which retains a plurality of coils therein, and at the top a plurality of individual coil-receiving, undercut grooves 90, an end tip receiver/protector undercut groove 92, and a connector handle receiver/protector 94, which also defines an undercut depression surrounded by raised embossed ridges at 93 to snugly retain the connector handle 95 therein.

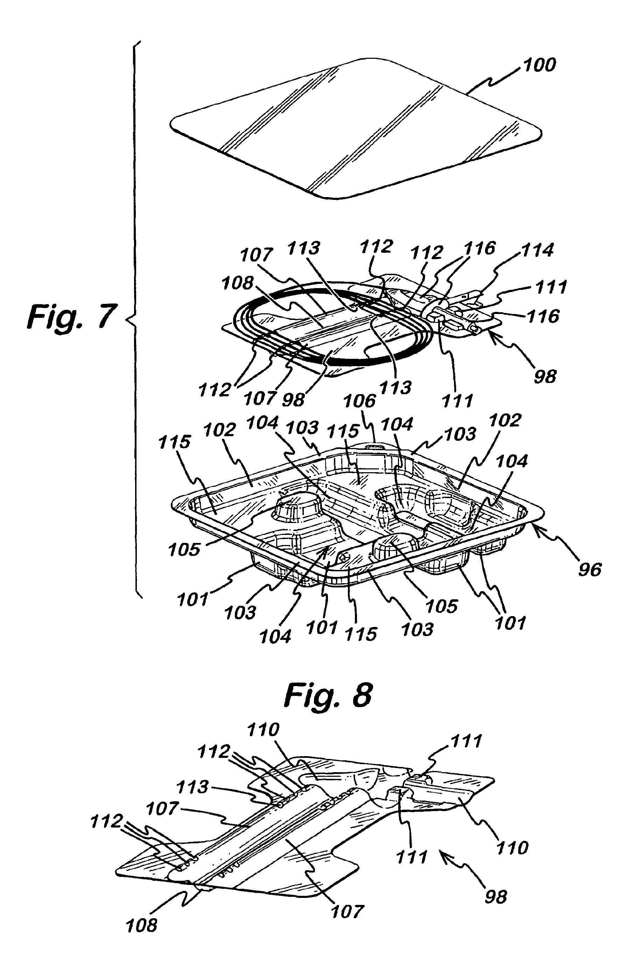

Figures 7 is an exploded view of a preferred embodiment of a combined packaging tray 96 and fiberoptic coil carrier 98 designed to secure and package a coil of fiberoptic cable, wherein the top portion of Figure 7 illustrates a separated Tyvek lid 100, the middle portion of Figure 7 illustrates just the fiberoptic coil carrier 98 securing a coil of fiberoptic cable thereto, and the bottom portion of Figure 7 illustrates just the packaging tray 96.

Referring to Figure 7, the blister tray 96 has a rectangular form, and is preferably thermoformed from clear PETG plastic. The blister tray has a bottom surface 101, sidewalls 102, and a flange 103 at the top of and extending around the sidewalls. The bottom surface is generally flat with raised studs 105 and I-shaped relief areas 104 defining a depression to receive the I-shaped carrier 98. The relief areas accommodate the larger components of the fiberoptic cable such as the connector handle, and provide sufficient room and clearance to allow fingers to grasp and remove the carrier. The carrier 98 and fiberoptic coils are supported by plateau surfaces 115, with the raised studs 105 rising above the plateau surfaces 115, and the relief areas 104 being below the plateau surfaces 115. The tray is configured to create a stable horizontal base having the shape of a large I which does not tip or rock when the tray is placed on a tabletop or stacked in multiples. The raised studs 105 maintain the carrier in position within the tray and provide support for the Tyvek lid 100 which is sealed to the flange 103 extending around the upper perimeter of the sidewalls 102. One flange corner 106 is recessed slightly, to provide an unsealed corner piece of the Tyvek lid 100 which is suitable for grasping to pry the Tyvek lid away from the blister tray.

Figure 8 is a perspective view of just the fiberoptic coil carrier as shown in the middle of Figure 7 but without an optical fiberoptic coil positioned thereon and without a spring clip thereon, and illustrates more clearly the molded contour of the carrier. Referring to Figures 7 and 8, the carrier 98 is generally flat, and is preferably thermoformed from clear or white PETG or high-impact polystyrene plastic. The exterior profile and I-shape thereof is shaped to fit within the sidewalls 102 and studs 105 and on top of the plateau surface 115 of the blister tray. Possible configurations for the carrier 98 include a cross, a Y shape, or the general shape of the letter I, as shown in Figures 7 and 8. Raised ribs 107 and a channel 108 near the center of the profile provide rigidity for the carrier. The uppermost area of the I has an attachment means, shown as a spring clip 114, which provides for attaching the carrier to a surgical drape.

The connector handle end 116 of the fiberoptic coil is seated in an uppermost area of the carrier in a recess 110 which is shaped to generally match the profile of the connector. Two thermoformed posts 111 directly across from each other are formed with a negative profile to match the circular barrel of the connector, to retain the connector barrel in place therein. The carrier 98 retains the fiberoptic cable in a coiled configuration by multiple snap fit recesses 112 located in the ribs. Each snap fit comprises a series of three (3) offsets which are formed with a negative degree draft which matches the profile of the fiberoptic cable while allowing ample room for ease in the forming process. The lowermost snap-fit recess in the top portion of the carrier at 113 has a larger size in order to secure therein the tip protector assembled to the treatment end of the fiberoptic cable. The fiberoptic coil is maintained at a diameter which is sufficiently large to allow long-term storage of the fiberoptic cable without inducing stress which might degrade the fiberoptic cable strength.

The carrier 98 can be securely attached to a surgical drape by a spring clip 114 at the top portion of the carrier. The attachment at 114 is centered side-to-side with respect to the center of gravity of the assembled carrier and fiberoptic coil so that the carrier will not skew to one side when hanging from the attachment. The clip can be formed of a combination of sterilizable metal and plastic materials such as a spring device (BGR, part number 2115). Alternatively, the attachment of the carrier to a surgical drape can be by an adhesive area or strip which adheres to the surgical drape. In one embodiment, the top portion of the carrier can fold back upon itself to enclose a portion of the drape and lock together via a mating stud and a hole.

The packaging concept of the present invention provides increased ease of handling of coils of fiberoptic cable by retaining the fiberoptic coils at a plurality of separate areas along the length of the fiberoptic cable until it is ready to be used. Each of the connector and treatment ends of the fiberoptic coil are separately retained, and can be removed and handled separately, while the opposite end and/or excess length are retained and controlled in the carrier. The sterile device can be conveniently accessible to a surgeon by clipping the carrier onto the surgical drape of a patient.

The carrier 98 of Figure 7 can selectively incorporate therein features from the embodiments of Figures 1-6, particularly the carrier features of Figures 2, 4, 5 and 6.

The blister tray 96 with a sealed Tyvek lid 100 provides for sterilization of the carrier/fiberoptic coil assembly in the lid-sealed blister tray. This sterilization can be, for example, by ethylene oxide which is particularly suitable in the sealed tray, electron-beam radiation, gas plasma or pulsed light.

The tray 96 is not specific to a particular carrier for a particular surgical device, and can accommodate any standard sized fiberoptic coil carrier regardless of the features required to retain the fiberoptic coils. The exterior bottom of the tray 96 is designed to allow easy stacking of multiple trays in a stable manner without the stack becoming skewed or tipping over.

Each carrier 98 is generally designed and contoured to be specific to a particular surgical device, and is configured to retain the device until it is removed for usage. The carrier can be attached to the surgical drape of a patient, such that it is positioned conveniently to and requires a minimum of handling by the surgeon.

The entire package of carrier 98, tray 96 and Tyvek lid 100 is cost competitive with alternate package configurations which utilize multiple thermoformed blisters and/or pouches. The assembly secures a proper coil configuration for long term storage of the fiberoptic coil, and maintains the fragile working ends thereof in a stable, protected manner to prevent breakage during shipping, handling and removal.

While several embodiments and variations of the present invention for a fiberoptic coil tray and carrier package are described in detail herein, it should be apparent that the disclosure and teachings of the present invention will suggest many alternative designs to those skilled in the art.

## Claims

1. A packaging arrangement for a coil of fiberoptic cable which includes a plurality of individual coil loops, comprising:
a. an outer packaging tray; and
b. a fiberoptic coil carrier which is inserted into the outer packaging tray for shipment or storage, and which can be removed from the tray, wherein the carrier provides increased ease of handling of the fiberoptic coil by retaining it with a plurality of separate retainers along the length of the fiberoptic cable, such that a selected length of the fiberoptic cable can be removed from the carrier and remaining coils of the fiberoptic cable remain secured to the carrier.

2. The packaging arrangement of claim 1, wherein the carrier also defines a connector end retainer for retaining a connector end of the fiberoptic cable, and a treatment end retainer for retaining a treatment end of the fiberoptic cable.

3. The packaging arrangement of claim 1, wherein the outer packaging tray is sealed with a top closure, wherein the closure-sealed tray provides for sterilization of the carrier/fiberoptic coil assembly in the outer packaging tray.

4. The packaging arrangement of claim 1, wherein the carrier includes an attachment means for attaching the carrier to a support, such that a surgeon can position the carrier conveniently to require a minimum of handling.

5. The packaging arrangement of claim 4, wherein the attachment means comprises a spring clip.

6. The packaging arrangement of claim 1, wherein the carrier is formed from molded plastic, and includes a plurality of molded individual coil loop retainers, each of which retains and secures a single coil loop of the fiberoptic cable, which allows each loop to be individually released to eliminate springing, a molded retainer to retain and secure a distal tip of the fiberoptic cable, and a molded retainer to retain and secure a connector handle of the fiberoptic cable.

7. The packaging arrangement of claim 6, wherein each individual coil loop retainer is formed by a molded groove.

8. The packaging arrangement of claim 7, wherein each molded groove defines a pair of opposed undercut shoulders which snap around an inserted individual coil loop.

9. A method of packaging a coil of fiberoptic cable which includes a plurality of individual coil loops, comprising:
a. mounting the fiberoptic coil on a fiberoptic coil carrier which secures the fiberoptic coil to the carrier with a plurality of separate retainers along the length of the fiberoptic cable, such that a selected length of the fiberoptic cable can be removed from the carrier and remaining coils of the fiberoptic cable remain secured to the carrier;
b. packaging the fiberoptic coil carrier with the fiberoptic coil mounted thereon in an outer packaging tray.

10. The method of claim 9, further including sealing the outer packaging tray with a top closure, and sterilizing the carrier/fiberoptic coil assembly in the closure-sealed outer packaging tray.
